# EUROPEAN PATENT APPLICATION

(11) **EP 4 560 648 A1**
(43) Date of publication of application: **28.05.2025**
(21) Application number: 24213473.2
(22) Date of filing: 18.11.2024
(51) Int. Cl.: G16H 50/30, G16H 50/50

(54) **GENERATING SYNTHETIC TRAINING DATA**

(30) Priority: 22.11.2023 EP 23211497
(71) Applicant: Bayer AG, 51373 Leverkusen (DE)
(72) Inventor: Hooge, Jens, 13086 Berlin (DE); Mohammadi, Seyed Sadegh, 50968 Köln (DE); Cersovsky, Josef, 12051 Berlin (DE); Jimenez, Guillermo, 41006 Sevilla (ES); Louro Costa Osorio, Pedro, 08860 Castelldefels (ES); Montalt, Javier, 46014 Valencia (ES)
(74) Representative: BIP Patents

(57) **Abstract**

Systems, methods, and computer programs disclosed herein relate to generating synthetic training data for training machine learning models.

## Description

### FIELD OF THE DISCLOSURE

Systems, methods, and computer programs disclosed herein relate to generating synthetic training data for training machine learning models.

### BACKGROUND

Artificial intelligence is spreading rapidly in the healthcare field. Machine learning models are being used to diagnose diseases (see, e.g., WO2018202541A1), detect and identify tumor tissue (see, e.g., WO2020229152A1), reduce the amount of contrast media used in radiological examinations (see, e.g., WO2022184297A1), and speed up radiological examinations (see, e.g., WO2021052896A1).

For the development of machine learning models, training data is needed to train the models for the described tasks and/or additional/different tasks. The quality and performance of a machine learning model is largely determined by the training data: the model can only be as good as the training data; the model can only predict what it has been taught by means of the training data.

There are a number of issues that complicate the provision of training data in the healthcare field. Providing training data for machine learning models in healthcare requires studies on healthy people and on patients suffering from a disease; such studies are expensive and time-consuming. Medical image data in particular is expensive and/or complex to generate. Since patient data is personal data, high legal requirements apply in the area of data protection; for example, patients must consent to the use of their data to train machine learning models. Secondly, training machine learning models require expert annotations or disease indicators as targets for model predictions; however, annotations are scarce, expensive, and time-consuming to generate, and represent the main bottleneck in medical artificial intelligence. Thirdly, patient data are often not balanced; patient data are often not representative of all populations; there is a risk of bias. Finally, there is often too little data available, especially for rare diseases.

A commonly used technique to increase the size of a training dataset is data augmentation. In image augmentation, modifications of the available images are generated. However, the amount of variation in training data that can be achieved through augmentation is limited. Furthermore, modifications of medical images are still images that can be associated with a subject and are therefore subject to privacy protection.

So, there is a need for data that can be used to train machine learning models. It would be desirable to have training data that on the one hand reflects reality, but on the other hand is not subject to data privacy restrictions. It would be desirable to have sufficient data available at any time and any place to train machine learning models.

### SUMMARY

These problems are addressed by the subject matter of the independent claims of the present disclosure. Some embodiments are defined in the dependent claims, the description, and the drawings.

In a first aspect, the present disclosure relates to a computer-implemented method for generating synthetic training data, the method comprising the steps:
- providing a data set, the data set comprising a variety of non-annotated medical records, wherein each medical record of the variety of non-annotated medical records represents a patient or part thereof,
- for each medical record of the variety of non-annotated medical records: generating a semantic representation of the medical record,
- providing a generative model,
- training the generative model in a self-supervised training procedure using the variety of non-annotated medical records and their semantic representations as training data, wherein the generative model is trained to reconstruct each medical record or a portion thereof using the respective semantic representation of the medical record as a conditioning input,
- providing one or more prompts and/or reference medical records and/or noisy data,
- generating one or more synthetic medical records based on the one or more prompts and/or reference medical records and/or noisy data using the trained generative model,
- outputting the one or more synthetic medical records, storing the one or more synthetic medical records in a data storage, transmitting the one or more synthetic medical records to a separate computer system, and/or using the one or more synthetic medical records for training another machine learning model.

In another aspect, the present disclosure provides a computer system comprising:
a processor; and
a memory storing an application program configured to perform, when executed by the processor, an operation, the operation comprising:
   - providing a data set, the data set comprising a variety of non-annotated medical records, wherein each medical record of the variety of non-annotated medical records represents a patient or part thereof,
   - for each medical record of the variety of non-annotated medical records: generating a semantic representation of the medical record,
   - providing a generative model,
   - training the generative model in a self-supervised training procedure using the variety of non-annotated medical records and their semantic representations as training data, wherein the generative model is trained to reconstruct each medical record or a portion thereof using the respective semantic representation of the medical record as a conditioning input,
   - providing one or more prompts and/or reference medical records and/or noisy data,
   - generating one or more synthetic medical records based on the one or more prompts and/or reference medical records and/or noisy data using the trained generative model,
   - outputting the one or more synthetic medical records, storing the one or more synthetic medical records in a data storage, transmitting the one or more synthetic medical records to a separate computer system, and/or using the one or more synthetic medical records for training another machine learning model.

In another aspect, the present disclosure provides a non-transitory computer readable storage medium having stored thereon software instructions that, when executed by a processor of a computer system, receiving a set of class-labelled medical images,
- providing a data set, the data set comprising a variety of non-annotated medical records, wherein each medical record of the variety of non-annotated medical records represents a patient or part thereof,
- for each medical record of the variety of non-annotated medical records: generating a semantic representation of the medical record,
- providing a generative model,
- training the generative model in a self-supervised training procedure using the variety of non-annotated medical records and their semantic representations as training data, wherein the generative model is trained to reconstruct each medical record or a portion thereof using the respective semantic representation of the medical record as a conditioning input,
- providing one or more prompts and/or reference medical records and/or noisy data,
- generating one or more synthetic medical records based on the one or more prompts and/or reference medical records and/or noisy data using the trained generative model,
- outputting the one or more synthetic medical records, storing the one or more synthetic medical records in a data storage, transmitting the one or more synthetic medical records to a separate computer system, and/or using the one or more synthetic medical records for training another machine learning model.

### BRIEF DESCRIPTION OF THE DRAWINGS

Fig. 1 shows schematically by way of example a process of training a generative machine learning model.
Fig. 2 shows schematically by way of example another process of training a generative machine learning model.
Fig. 3 (a), 3 (b), and 3 (c) show how a control model is added to a pre-trained generative machine learning model.
Fig. 4 shows a schematic example of how a semantic representation can be generated from a medical record.
Fig. 5 shows a schematic example of how parts of a semantic representation can be masked.
Fig. 6 shows an embodiment of the computer-implemented method of generating synthetic training data in form of a flow chart.
Fig. 7 shows another embodiment of the computer-implemented method of generating synthetic training data in form of a flow chart.
Fig. 8 shows a schematic example of the generation of several semantic representations and the combination of the semantic representations into a single semantic representation.
Fig. 9 shows schematically an embodiment of the method of generating synthetic training data using the trained generative model.
Fig. 10 shows schematically another embodiment of the method of generating synthetic training data using the trained generative model.
Fig. 11 illustrates a computer system according to some example implementations of the present disclosure.

### DETAILED DESCRIPTION

Various example embodiments will be more particularly elucidated below without distinguishing between the aspects of the disclosure (method, computer system, computer-readable storage medium). On the contrary, the following elucidations are intended to apply analogously to all the aspects of the disclosure, irrespective of in which context (method, computer system, computer-readable storage medium) they occur.

If steps are stated in an order in the present description or in the claims, this does not mean that the disclosure is restricted to the stated order. On the contrary, it is conceivable that the steps can also be executed in a different order or else in parallel to one another, unless, for example, one step builds upon another step, this requiring that the building step be executed subsequently (this being, however, clear in the individual case). The stated orders are thus exemplary embodiments of the present disclosure.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides means for generating synthetic training data.

The term "synthetic" means that the training data is not the result of physical measurements on real objects/subjects, but that the training data has been generated by a generative model. A synonym for the term "synthetic" is the term "artificial".

The synthetic training data may be synthetic images, synthetic sound recordings (e.g., sound recordings of coughing, snoring, sneezing, hiccups, vomiting, screaming, swallowing, wheezing, shortness of breath, chewing, teeth grinding, chills, cramps, spasms, heartbeats), synthetic electrocardiograms, synthetic electroencephalograms, synthetic blood pressure values, synthetic temperature values, synthetic heartbeat rates, synthetic respiratory rates, synthetic blood glucose values, and/or synthetic data of other physiological and/or anatomical and/or laboratory values.

In an embodiment of the present disclosure, the synthetic training data is or comprises a synthetic signal caused by a body action.

"Body actions" are measurable events that can provide information about a patient's health status. Examples of body actions include cough, snoring, sneezing, hiccups, vomiting, shouting, swallowing, wheezing, shortness of breath, chewing, teeth grinding, chills, convulsions, spasm, heartbeat, respiration, electrical nerve impulses, and/or the like. A body action can be caused by an internal stimulus, or it can be a reaction to an external stimulus. A body action can be an unconscious or a conscious action. A body action can be intentional or unintentional (such as a reflex).

A "signal" is a function that conveys information about a phenomenon and/or an event. For example, a signal may be a change in a physical quantity over time. A signal can also be considered as a representation of a body action. For example, coughing produces sound that can be detected as an acoustic signal (sound signal). At the same time, coughing leads to movements of at least the upper body, which can be detected, e.g., as a visible signal by a camera and/or as an electrical signal by means of acceleration sensors. In addition, cough produces electromyographic signals.

The way in which a signal is generated, present and/or detected is also referred to as the "modality" of the signal. To stay with the example of coughing, a cough event usually generates an acoustic signal, an acceleration signal, an electrocardiogram signal, and other metrologically detectable signals that can be used for detection, identification and/or characterization of the event causing and/or accompanying the signals. The acoustic signal, the acceleration signal and the electromyographic signal are examples of signals of different modalities.

In an embodiment of the present disclosure, the signal is a sound signal (also referred to as audio signal), an electromyographic signal, an electrocardiogram signal, an accelerometer signal, a chest impedance signal, a temperature signal, and/or a plethysmographic signal.

In another embodiment of the present disclosure, the synthetic training data is or comprises an electroencephalogram.

In another embodiment of the present disclosure, the synthetic training data is or comprises blood pressure values over time.

In another embodiment of the present disclosure, the synthetic training data is or comprises a synthetic medical image. For the sake of simplicity, the aspects of the present disclosure are described in more detail with reference to synthetic images, but without intending to limit the disclosure to such synthetic data.

The term "image" as used herein means a data structure that represents a spatial and/or temporal distribution of a physical signal. The distribution may be of any dimension, for example 1D, 2D, 3D, 4D or any higher dimension. The distribution may be of any shape, for example forming a grid and thereby defining pixels or voxels, the grid being possibly irregular or regular. The physical signal may be any signal, for example proton density, tissue echogenicity, tissue radiolucency, measurements related to blood flow, information of rotating hydrogen nuclei in a magnetic field, color, level of gray, depth, surface or volume occupancy, such that the image may be a 2D or 3D RGB/grayscale/depth image, or a 3D surface/volume occupancy model. An image is usually composed of discrete image elements (e.g., pixels for 2D images, voxels for 3D images, doxels for 4D images).

In an embodiment of the present disclosure, the synthetic image is a synthetic medical image.

A "medical image" is a visual representation of the human body or a part thereof or a visual representation of the body of an animal or a part thereof. Medical images can be used, e.g., for diagnostic and/or treatment purposes. A widely used format for digital medical images is the DICOM format (DICOM: Digital Imaging and Communications in Medicine). There are of course many other image file formats (see, e.g., https://developer.mozilla.org/en-US/docs/Web/Media/Formats/Image_types) and the present disclosure is not limited to any specific image file format.

Techniques for generating medical images include X-ray radiography, computerized tomography, fluoroscopy, magnetic resonance imaging, ultrasonography, endoscopy, elastography, tactile imaging, thermography, microscopy, positron emission tomography, optical coherence tomography, fundus photography, and others.

Examples of medical images include CT (computer tomography) scans, X-ray images, MRI (magnetic resonance imaging) scans, PET (positron emission tomography) images, fluorescein angiography images, OCT (optical coherence tomography) scans, histological images, ultrasound images, fundus images and/or others.

In an embodiment, the medical image is a microscopic image, such as a whole slide histological image of a tissue of a human body. The histological image can be an image of a stained tissue sample. One or more dyes can be used to create the stained image. Usual dyes are hematoxylin and eosin.

In another embodiment, the medical image is a radiological image. "Radiology" is the branch of medicine concerned with the application of electromagnetic radiation and mechanical waves (including, for example, ultrasound) for diagnostic, therapeutic and/or scientific purposes. In addition to X-rays (radiography), other ionizing radiation such as gamma rays or electrons are also used. Since a primary purpose is imaging, other imaging procedures such as sonography and magnetic resonance imaging (MRI) are also included in radiology, although no ionizing radiation is used in these procedures. Thus, the term "radiology" as used in the present disclosure includes, in particular, the following examination procedures: radiography, computed tomography, magnetic resonance imaging, sonography, positron emission tomography.

The radiological image can be, e.g., a 2D or 3D CT scan or MRI scan. The radiological image may be an image generated using a contrast agent or without a contrast agent. It may also be multiple images, one or more of which were generated using a contrast agent and one or more of which were generated without a contrast agent.

Synthetic training data are generated using a generative model. A "generative model" is a type of machine learning model that is designed to learn and generate new data that resembles the training data it was trained on. Generative models capture the underlying distribution of the training data and can generate samples from that distribution.

The term "machine learning model", as used herein, may be understood as a computer implemented data processing architecture. The machine learning model can receive input data and provide output data based on that input data and on parameters of the machine learning model (model parameters). The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data usually contains the correct answer, which is referred to as the target. The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training, where the loss function can quantify the deviations between the output and the target. The loss function may be chosen in such a way that it rewards a wanted relation between output and target and/or penalizes an unwanted relation between an output and a target. Such a relation can be, e.g., a similarity, or a dissimilarity, or another relation.

A loss function can be used to calculate a loss for a given pair of output and target. The aim of the training process can be to modify (adjust) parameters of the machine learning model in order to reduce the loss to a (defined) minimum.

A machine learning model of the present disclosure can comprise several machine learning models, which are also referred to as submodels.

In the case of the present disclosure, a machine learning model is trained to generate synthetic training data.

The training of the machine learning model may include one or more phases.

It is possible that the machine learning model is trained in a first phase with non-annotated medical records and fine-tuned in a second phase with annotated medical records.

However, it is also possible that the machine learning model is only trained based on non-annotated medical records.

In a first step, a data set is provided. The data set comprises a variety of non-annotated medical records. Each medical record of the variety of non-annotated medical records represents a patient or part thereof or a signal caused by a body action of the patient or other anatomical and/or physiological characteristics of the patient.

Each patient is a living being, e.g., an animal (e.g., a mammal) or a human being. In an embodiment of the present disclosure, each patient is a human.

Each medical record may be an image, a sound recording (e.g., sound recordings of coughing, snoring, sneezing, hiccups, vomiting, screaming, swallowing, wheezing, shortness of breath, chewing, teeth grinding, chills, cramps, spasms, heartbeats), an electrocardiogram, an electroencephalogram, one or more blood pressure values, one or more temperature values, one or more heartbeat rates, one or more respiratory rates, one or more blood glucose values, and/or one or more other physiological and/or anatomical and/or laboratory values.

The data in the medical records may be or comprise measured data.

In an embodiment of the present disclosure, the medical records are medical images. Each medical image may represent a patient or a portion thereof. The medical images can be images of different modalities.

The medical images can include, for example, X-ray images, CT images, MRI images, ultrasound images and/or PET images.

The medical images can include, for example, optical coherence tomography (OCT) images, fluorescein angiography (FA) images, infra-red reflectance (IR) images, infra-red autofluorescence (IR-AF) images, near-infra-red autofluorescence (NIR-AF) images, confocal scanning laser ophthalmoscopy (cSLO) images, fundus autofluorescence (FAF) images, color fundus photography (CFP) images, OCT-angiography images, fluorescence life-time imaging ophthalmology (FLIO) images, fluorescence lifetime imaging (FLIM) images, and/or polarized fluorescence microscopy images.

The medical images can include, for example, microscopic images of tissue samples. The medical images may be microscopic images at various magnifications. The medical images may be whole slide histological image of a tissue of a human and/or animal body. The medical images may be images of stained tissue samples. One or more dyes can be used to create stained images; usual dyes are hematoxylin and eosin. The tissue sample may be from any source in the subject's body including, but not limited to, skin (including portions of the epidermis, dermis, and/or hypodermis), bone, bone marrow, brain, thymus, spleen, small intestine, appendix, colon, rectum, liver, gall bladder, pancreas, kidney, lung, ureter, bladder, urethra, uterus, ovary, cervix, scrotum, penis, prostate.

The medical images can represent one or more (e.g., different) examination areas of a plurality of patients.

The examination area can be or comprise a thorax, breast, stomach, liver, kidney, heart, lung, brain, stomach, bladder, prostate, intestine, eye, or a part of said parts or another part of the body of a mammal (for example a human).

In a further step, a semantic representation of the medical record is generated for each medical record of the variety of non-annotated medical records.

A "semantic representation" of a medical record refers to a mathematical or computational representation that captures the meaning or semantics of the medical record. It aims to encode the high-level information and concepts present in the medical record, allowing machines to understand and reason about the content of the medical record.

In the case of an image, for example, information about morphologies, colors, structures and/or relationships between structures contained in the image can be agglomerated in a semantic representation of the image.

Semantic representations can be learned through training on large datasets using techniques such as deep learning.

Convolutional neural networks (CNNs) are frequently used for generating semantic representations, in particular for images. These artificial neural networks consist of multiple layers that progressively extract features at different levels of abstraction, capturing both low-level details and higher-level semantic concepts. The learned semantic representation of a medical record may be a vector or a set of numerical values that encodes the characteristics and semantic information of the medical record. This representation can be used for various tasks, such as classification, regression, segmentation, information retrieval, and/or other/further tasks.

By learning semantic representations, machines can associate medical records such as images with meaningful labels or concepts, enabling them to perform tasks that require understanding and reasoning about their content. For example, semantic representations play a crucial role in bridging the gap between raw pixel data and higher-level understanding in the field of computer vision and machine learning.

In an embodiment of the present disclosure, the semantic representations of the medical records are embeddings generated with an encoder of a pre-trained autoencoder.

An "embedding" is a numerical representation of a medical record, usually in the form of a vector or matrix. In such an embedding, such features of a medical record can be summarized that make up the content of the medical record. Thus, the embedding can be a compressed representation of the medical record.

An "autoencoder" is a type of neural network architecture that is primarily used for unsupervised learning and dimensionality reduction. It is designed to learn a compressed representation, or embedding, of the input data and then reconstruct the original data from this embedding. An autoencoder usually comprises two main components: an encoder and a decoder. The encoder takes the input data and maps it to a lower-dimensional latent space representation, also known as the embedding or "latent". The decoder then takes this embedding and reconstructs the original input data from it. The objective of an autoencoder is to minimize the reconstruction error, which encourages the model to learn a compressed representation that captures the most salient features of the input data.

An autoencoder is often implemented as an artificial neural network that comprises a convolutional neural network (CNN) to extract features from medical images as input data. An example of such an autoencoder is the U-Net (see, e.g., O. Ronneberger et al.: U-net: Convolutional networks for biomedical image segmentation, International Conference on Medical image computing and computer-assisted intervention, 234-241, Springer, 2015, DOI: 10.1007/978-3-319-24574-4_28). Further examples of autoencoders are sparse autoencoders, denoising autoencoders, variational autoencoders (VAEs), and generative adversarial networks (GANs). The autoencoder can be (pre-)trained based on (non-annotated) images. The images can be medical images, but they can also be other images or include other images.

Autoencoders can be (pre-)trained using a self-supervised learning approach, meaning they do not require labeled data for training.

The term "pre-trained" refers to a model that has been trained on a large dataset in advance and is then made available (e.g., for finetuning). Pre-training involves training a model on a task or dataset that is typically different from the specific task for which the model will be used later. The pre-training process involves exposing the model to a vast amount of data and allowing it to learn general patterns and representations from that data. This enables the model to capture common features and structures that are useful across various related tasks. The model is typically trained using unsupervised or self-supervised learning methods, where the labels or annotations are generated automatically and/or do not require human intervention. Once the pre-training phase is complete, the model's weights and parameters are saved and made publicly available. Other researchers or practitioners can then use this pre-trained model as a starting point for their own tasks. By leveraging the pre-trained model, they can benefit from the learned representations and potentially achieve better performance even with limited training data.

In another embodiment of the present disclosure, the semantic representations of the medical records are embeddings generated with the help of a pre-trained transformer.

Transformers are widely used for various natural language processing tasks, including machine translation, text summarization, sentiment analysis, and more.

At the core of the transformer model is the transformer architecture, which relies heavily on attention mechanisms to process sequential data efficiently. Unlike traditional recurrent neural networks (RNNs) or convolutional neural networks (CNNs), transformers do not employ recurrent or convolutional operations. Instead, they use attention mechanisms to capture contextual relationships between words or tokens in a sequence. This enables transformers to model long-range dependencies effectively, allowing them to produce more accurate predictions.

The transformer architecture consists of two main components: the encoder and the decoder. The encoder processes the input sequence, modeling its contextual relationships, while the decoder generates the output sequence based on the encoded information. Both the encoder and decoder are composed of multiple layers of attention mechanisms and feed-forward neural networks. The attention mechanism allows the model to focus on different parts of the input sequence while considering the dependencies between tokens.

Transformers have significantly contributed to advancements in machine learning, particularly in natural language processing tasks. Their ability to capture contextual information efficiently has resulted in state-of-the-art performance on various benchmarks and has paved the way for numerous applications in the field (see, e.g., T. Lin et al.: A survey of transformers, AI Open, Volume 3, 2022, Pages 111-132).

In a vision transformer, the input image is divided into a sequence of patches, which are then flattened and fed into a series of transformer layers. These transformer layers may consist of attention modules and feed-forward neural networks. The attention mechanism allows the model to capture the relationships between different patches and learn global context information, while the feed-forward networks enable non-linear transformations (see, e.g., S. Khan et al.: Transformers in Vision: A Survey, arXiv:2101.01169v5).

The key advantage of vision transformers is their ability to model long-range dependencies and capture global context, which is crucial for understanding complex visual patterns and relationships.

Like the autoencoder, the vision transformer is pre-trained. The vision transformer may have been pre-trained in a supervised, self-supervised or unsupervised approach.

The vision transformer may have been pre-trained in a DINO approach. DINO (self-DIstillation with NO labels) is a self-supervised learning method specifically designed to improve the performance of vision transformers in image classification tasks (see, e.g., M. Caron et al.: Emerging Properties in Self-Supervised Vision Transformers, arXiv:2104.14294v2).

"Self-supervised learning" is a type of machine learning paradigm where a model is trained to learn from the data itself, without the need for human-labeled annotations. Instead of relying on external labels provided by humans, the model generates its own supervisory signals from the input data, making it a form of unsupervised learning.

In traditional self-supervised learning, a model can be trained on a first task (e.g., an auxiliary task), where the labels are generated from the input data itself without requiring human annotations. The model learns to predict certain properties or relationships within the data, which in turn helps it to learn meaningful representations. These representations can then be transferred to downstream tasks.

DINO introduces a novel approach to self-supervised learning for vision transformers by leveraging two main components: clustering and distillation. Initially, the model is trained to cluster the augmented views of the input data. This clustering helps the model to discover semantically similar instances within the dataset. Then, a distillation process is performed, where the model learns to transfer knowledge from a teacher network to a student network. The teacher network provides soft targets, or guidance, to the student network, which helps improve the student's performance. By combining clustering and distillation, DINO enables the model to learn more robust and discriminative representations, leading to better generalization and performance on downstream tasks such as image classification.

In another embodiment of the present disclosure, the vision transformer is pre-trained using a DiNOv2 approach. DiNOv2 (Discriminative NOise Contrastive Learning V2) is another self-supervised approach for training vision transformers (see, e.g., M. Oquab et al.: DINOv2: Learning Robust Visual Features without Supervision, arXiv:2304.07193v1).

In another embodiment of the present disclosure, the semantic representations of the medical images are embeddings generated with the help of an image encoder of a pre-trained CLIP model.

CLIP (Contrastive Language-Image Pretraining) is a framework in the field of machine learning that combines natural language processing and computer vision to understand and generate multimodal representations of images and text. CLIP encodes text and image in same embedding space (see, e.g., A. Radford et al.: Learning Transferable Visual Models From Natural Language Supervision, arXiv:2103.00020v1).

CLIP is (pre-)trained in a self-supervised manner, where large-scale datasets of images and their associated text are used to learn joint representations. The model is trained to associate images and their textual descriptions by maximizing their similarity in the learned embedding space. This allows CLIP to understand and reason about images and text in a shared semantic space. The base model uses a ViT-L/14 transformer architecture as an image encoder and uses a masked self-attention transformer as a text encoder. These encoders are trained to maximize the similarity of (image, text) pairs via a contrastive loss.

The key innovation of CLIP is its ability to generalize across different domains and tasks. By training on a diverse range of image and text pairs, CLIP can perform a variety of tasks without task-specific fine-tuning. For example, CLIP can perform zero-shot image classification, where it can classify images into categories it has never seen during training, solely based on textual descriptions.

In an embodiment, the image encoder of a pre-trained CLIP model is used which was pre-trained on medical images, such as BiomedCLIP (see, e.g., S. Zhang et al.: Large-Scale Domain-Specific Pretraining for Biomedical Vision-Language Processing, arXiv:2303.00915v1).

However, the models and training procedures listed here are only examples; the semantic representations of the medical records can also be generated in other ways.

In a next step, a generative model is provided. The generative model is a type of machine learning model. The generative model is configured to generate a synthetic medical record based on input data.

In an embodiment of the present disclosure, the generative model is a diffusion model.

Diffusion models focus on modeling the step-by-step evolution of data distribution from a "simple" starting point to a "more complex" distribution. The underlying concept of diffusion models is to transform a simple and easily sampleable distribution, typically a Gaussian distribution, into a more complex data distribution of interest. This transformation is achieved through a series of invertible operations. Once the model learns the transformation process, it can generate new samples by starting from a point in the simple distribution and gradually "diffusing" it to the desired complex data distribution.

A diffusion model usually comprises a noising model and a denoising model.

The noising model usually comprises a plurality of noising stages. The noising model is configured to receive input data (e.g., an image) and produce noisy data in response to receipt of the input data. The noising model introduces noise to the input data to obfuscate the input data after a number of stages, or "timesteps" *T.* The noising model can be or can include a finite number of steps *T or* an infinite number of steps (*T*→ ∞). The noising model may have the same weights/architectures for all timesteps or different weights/architectures for each timestep. The number of timesteps can be global (i.e., timesteps are the same for all pixels of an image) or local (e.g., each pixel in an image might have a different timestep).

The denoising model is configured to reconstruct the input data from the noisy data. The denoising model is configured to produce samples matching the input data after a number of stages.

For example, the diffusion model may include Markov chains at the noising model and/or denoising model. The diffusion models may be implemented in discrete time, e.g., where each layer corresponds to a timestep. The diffusion model may also be implemented in arbitrarily deep (e.g., continuous) time.

For example, the diffusion model may be fully Gaussian such that an unbiased estimate of the objective function can be obtained from a single layer. It is thus possible to avoid computing intermediate layers.

Diffusion models can be conceptually similar to a variational autoencoder (VAE) whose structure and loss function provides for efficient training of arbitrarily deep (e.g., infinitely deep) models. The diffusion model can be trained using variational inference, for example.

The diffusion model can be a Latent Diffusion Model (LDM). In such a model, the diffusion approach in the case of an image is not performed in pixel space, but in so-called latent space based on a representation of the image, usually a compressed representation (see, e.g., R. Rombach et al.: High-Resolution Image Synthesis with Latent Diffusion Models, arXiv:2112.10752v2).

The diffusion model may be a Denoising Diffusion Probabilistic Model (DDPM). DDPMs are a class of generative models that work by iteratively adding noise to input data (e.g., an image or a compressed representation) and then learning to denoise from the noisy signal to generate new samples (see, e.g., J. Ho et al.: Denoising Diffusion Probabilistic Models, arXiv:2006.11239v2).

The diffusion model may be a Score-based Generative Model (SGM). In SGMs the data is perturbed with random Gaussian noise of various magnitudes. With the gradient of log probability density as score function, samples are generated towards decreasing noise levels and the model is trained by estimating the score functions for noisy data distribution (see, e.g., Y. Song et al.: Score-Based Generative Modeling through Stochastic Differential Equations, arXiv:2011.13456v2).

The diffusion model may be a Denoising Diffusion Implicit Model (DDIM) (see, e.g.: J. Song et al.: Denoising Diffusion Implicit Models, arXiv:2010.02502v4). A critical drawback of DDPMs is that they require many iterations to produce a high-quality sample. For DDPMs, this is because the generative process (from noise to data) approximates the reverse of the forward diffusion process (from data to noise), which could have thousands of steps; iterating over all the steps is required to produce a single sample. DDIMs are implicit probabilistic models that are closely related to DDPMs, in the sense that they are trained with the same objective function. DDIMs allow for much faster sampling while keeping an equivalent training objective. They do this by estimating the addition of multiple Markov chain steps and adding them all at once. DDIMs construct a class of non-Markovian diffusion processes which makes sampling from reverse process much faster. This modification in the forward process preserves the goal of DDPM and allows for deterministically encoding an image to the noise map.

Unlike DDPMs, DDIMs enable control over image synthesis owing to the latent space flexibility (attribute manipulation) (see, e.g., K. Preechakul et al.: Diffusion autoencoders: Toward a meaningful and decodable representation, arXiv:2111.15640v3). With DDIM, it is possible to run the generative process backward deterministically to obtain the noise map x*_{T}*, which represents the latent variable or encoding of a given image x₀. In this context, DDIM can be thought of as an image decoder that decodes the latent code x*_{T}* back to the input image. This process can yield a very accurate reconstruction; however, x*_{T}* still does not contain high-level semantics as would be expected from a meaningful representation.

In an embodiment of the present disclosure, the diffusion model is a conditional diffusion model.

In a conditional diffusion model, a condition is used to denoise latent data and reconstruct the input data (see, e.g, P. Dhariwal, A. Nichol: *"Diffusion models beat GANs on image synthesis,"* arXiv:2105.05233v4). One benefit of conditioning the diffusion model with information-rich representations is a more efficient denoising process.

In general, such a condition can be based on a text (e.g., text-to-image), on an image, on audio data, or on other information. According to the invention, the semantic representation of the medical record is used as a condition for reconstructing the medical record.

In a next step, the generative model is trained in a self-supervised training procedure using the variety of non-annotated medical records and their semantic representations as training data, wherein the generative model is trained to reconstruct each medical record or a portion thereof using the respective semantic representation of the medical record as a conditioning input.

The training may take place in an unsupervised or self-supervised training procedure. No annotated medical records are required for training. This enables automated training on a large training data set.

Instead of an annotation (label), a semantic representation of each medical record is used during training. So, for each medical record of the variety of medical records, a semantic representation of the medical image is generated. The semantic representation serves as a conditioning input during training. The generative model is trained to reconstruct each medical record using the respective semantic representation of the medical record as a conditioning input.

Masking techniques can be used during training. For example, it is possible to mask part of the semantic representations. Masking forces the conditional generative model to compensate for the missing information. It learns to extract global information from local information.

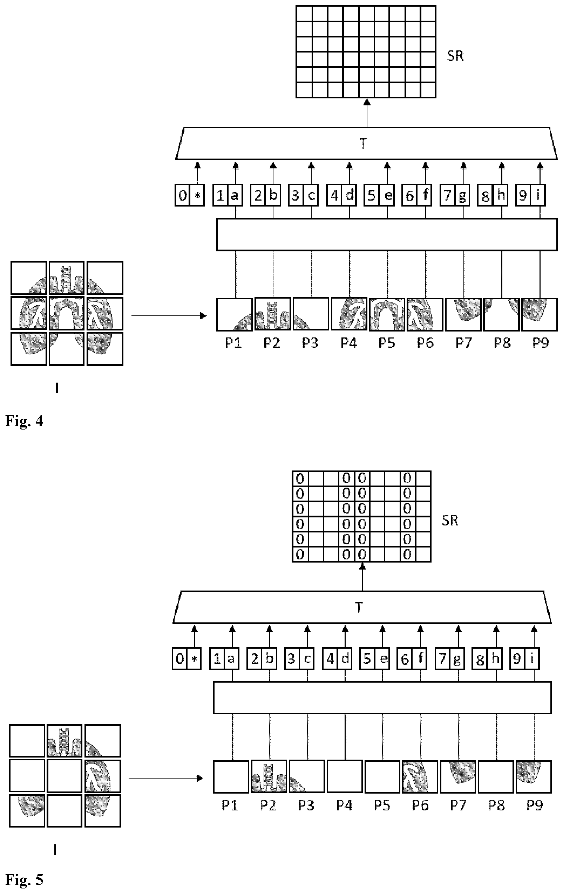

The parts that are masked can be selected randomly or specifically. The proportion of masked parts can be constant or varied. An example is shown in Fig. 5.

The training of the generative model can be terminated if one or more stop criteria are met. Such stop criteria can be for example: a predefined maximum number of training steps has been performed, deviations between output data and target data can no longer be reduced by modifying model parameters, a predefined minimum of the loss function is reached, and/or an extreme value (e.g., maximum or minimum) of another performance value is reached.

If the generative model is trained on medical images, it may be useful to train the generative model on many different medical images from a range of modalities.

However, it is also possible in principle to train the generative model based on different modalities (not just images, see, e.g.: WO2022184516A1, WO2022228958A1, WO2023011943A1).

The described training with the non-annotated medical records (first training phase) can be followed by a second training phase.

The first training phase may be about training the generative model on a first set of non-annotated training data to gain a general understanding of medical records, e.g., what structures occur and/or what short-, medium- and long-term correlations are present.

In a second training phase, the generative model can be fine-tuned. Fine-tuning can be carried out, for example, with the help of training data based on annotated medical records.

Fine-tuning with the annotated medical records can be carried out in the same way as training with the non-annotated medical records. The annotations can be used as an additional condition when reconstructing the medical records.

The data set with annotated medical records for fine-tuning the generative model can be smaller than the first data set with non-annotated medical records.

In an embodiment of the present disclosure, a control model is used to control the fine-tuning. This is also known as indirect fine-tuning.

The direct finetuning or continued training of a large pretrained model with limited data may cause overfitting and/or catastrophic forgetting (see, e.g., N. Ruiz et al.: DreamBooth: Fine Tuning Text-to-Image Diffusion Models for Subject-Driven Generation, arXiv:2208.12242v2). A control model can remedy this. The control model is added to the (pre-)trained generative model. The control model may add further condition control to the (pre-)trained generative model.

A "control model" may be a neural network structure to generative models by adding extra conditions. It may copy the weights of neural network blocks of the trained generative model into a "locked" copy and a "trainable" copy. The "trainable" copy learns one or more further conditions. The "locked" copy preserves the trained generative model. The neural architecture may be connected with "zero convolutions" (zero-initialized convolution layers) that progressively grow the parameters from zero and ensure that no harmful noise could affect the fine-tuning.

Such a control model is described in the scientific literature, for example, under the term "ControlNet" (see, e.g.; L. Zhang et al.: Adding Conditional Control to Text-to-Image Diffusion Models, arXiv:2302.05543v2).

It is also possible to freeze the (pre-)trained generative model weights and inject trainable rank decomposition matrices into each layer of the transformer architecture. This allows to train some dense layers in the generative model indirectly by optimizing rank decomposition matrices of the dense layers' change during adaptation instead, while keeping the (pre-)trained weights frozen (see, e.g., E. Hu et al.: LoRa: Low-Rank Adaptation of Large Language Models, arXiv:2106.09685v2).

The control model added to the trained generative model is trained in a second training with a second data set. The second data set comprises annotated (labelled) medical records. In other words: for each medical record an annotation is available.

The annotation may provide information about what is contained in the medical record and/or which patient and/or which part of a patient the medical record represents and/or what the medical record represents and/or how the medical record was created/acquired and/or other/additional information about the medical record and/or the content of the medical record and/or the creation/acquisition of the medical record.

An annotation can be a text; however, it does not necessarily have to be a text. It can also be other data. The annotations only need to have a relationship to the medical records so that the annotation changes when the medical record changes.

For example, it is conceivable that the medical record is a CT scan of a patient's examination area and the annotation is an MRI scan of the patient's examination area, or *vice versa.*

It is possible that the medical record is a radiologic image representing an examination area of a patient after the application of a first amount of a contrast agent, while the annotation represents the examination area of the patient after the application of a second amount of the contrast agent, wherein the first amount and the second amount are different, wherein the first amount or the second amount may be zero.

It is possible that the medical record is a radiologic image representing an examination area of a patient at a first time point before or after the application of a contrast agent, while the annotation represents the examination area of the patient at a second time point before or after the application of the contrast agent, wherein the first time point and the second time point are different.

It is possible that the medical record is a radiological image of a patient's brain or part of the brain, while the annotation is an electroencephalogram of the patient.

It is possible that the medical record is a radiologic image of a patient's lung or part of the lung, while the annotation is an audio recording of one or more of the patient's coughing events.

It is possible that the medical record is a microscopic image of a tissue sample of a patient and the annotation comprises a gene sequence or protein sequence of the tissue sample.

Many other possibilities are conceivable.

From each annotation, a semantic representation may be generated. To generate a semantic representation of an annotation, the methods for generating a semantic representation of a medical record described above can be used.

For example, if the annotation is a text, a semantic annotation representation can be generated by a text encoder, e.g., a text encoder of a CLIP model, or any other word embedding technique (see, e.g., S. Selva Birunda and R. Kanniga Devi: A Review on Word Embedding Techniques for Text Classification in Innovatove Data Communication Technologies and Application, Proceedings of ICIDCA 2020, Vol. 59, Springer, ISSN 2367-4512, 267-281; M. T. Pilehvar, J. Camacho-Collados: Embeddings in Natural Language Processing, Morgan&Claypool Publishers 2021, ISBN: 9781636390222).

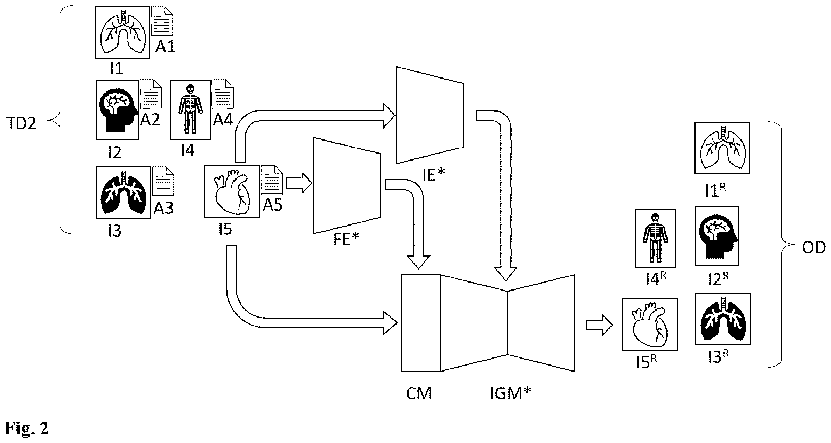

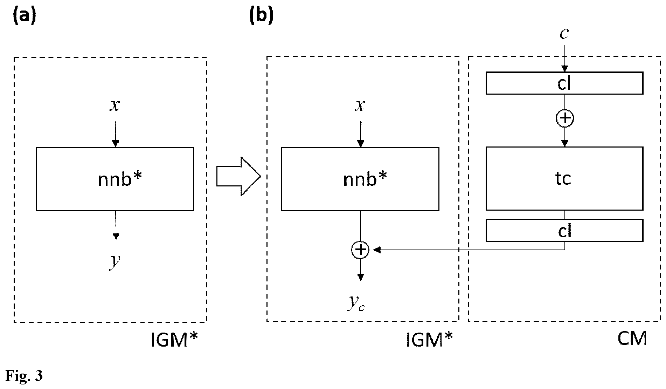

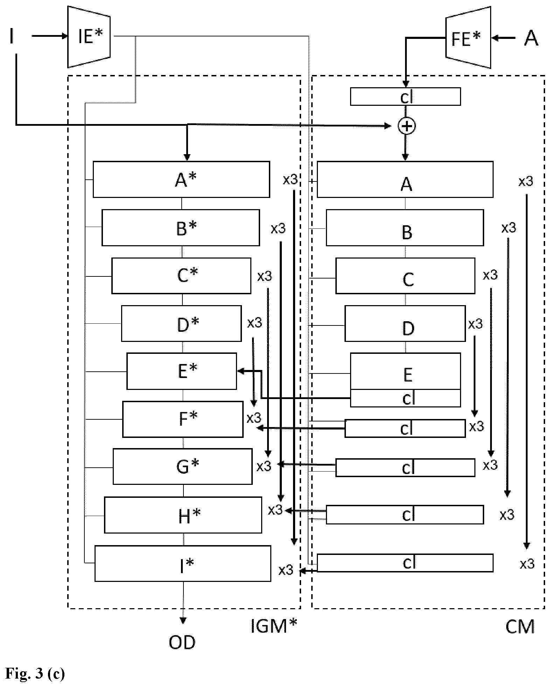

The semantic annotation representations may be used as conditions for reconstructing the respective medical record. The training procedure is described in more detail below with reference to Figs. 2, 3 (a), 3 (b), and 3 (c) using medical images as an example.

The training can be terminated if one or more stop criteria are met. Such stop criteria can be for example: a predefined maximum number of training steps has been performed, deviations between output data and target data can no longer be reduced by modifying model parameters, a predefined minimum of the loss function is reached, and/or an extreme value (e.g., maximum or minimum) of another performance value is reached.

The trained machine learning model (e.g., the trained generative model comprising the trained control model) can be output after training, stored, transferred to a separate computer system and/or used to generate synthetic medical images.

In a further phase, the trained machine learning model can be used to generate one or more synthetic medical records.

A synthetic medical record may be generated based on a prompt and/or a reference medical record and/or noisy data.

A prompt corresponds to an annotation, i.e., the prompt may provide additional information about what the synthetic medical record should represent. A prompt usually has the same modality as the annotations that were used to fine-tune the generative model.

A prompt can be a text prompt, for example. A synthetic medical image can be generated based on such a text prompt and/or based on a reference image.

The text prompt can specify what kind of image it should be (e.g., X-ray, CT, MRI, ultrasound, PET, OCT, FA, IR, IR-AF, NIR-AF, cSLO, FAF, CFP, FLIO, FLIM, microscopic or other), which measurement protocol it should correspond to, which examination area it should show, in which magnification the examination area should be shown, whether the examination area should be shown before or after the application of a quantity of contrast medium, how large the quantity of contrast medium should be, if applicable, which examination object it should be (e.g. human, animal, male, female, age, height, weight), which dye was used to color the tissue sample, if applicable, which disease the examination object may be suffering from, and/or other/additional specifications.

From such a text prompt, a semantic representation of the text prompt can be generated, e.g., by means of the same text encoder which was used to generate semantic annotation representations of the annotated medical images of the second training data set. Such a semantic representation of the text prompt can be entered into the trained machine learning model. The model generates a synthetic medical image based on the semantic representation of the text prompt and noisy data, which can be random, for example.

Instead of a text prompt and/or in addition to a text prompt, a reference image can (also) be used to generate a synthetic medical image.

Like the text prompt, the reference image can specify what kind of image the synthetic image should be, which measurement protocol it should correspond to, which examination area it should show, in which magnification the examination area should be shown, whether the examination area should be shown before or after the application of a quantity of contrast medium, how large the quantity of contrast medium should be, if applicable, which examination object it should be, which dye was used to color the tissue sample, if applicable, which disease the examination object may be suffering from, and/or other/additional specifications.

A reference image can be selected for this purpose, which is a respective image type, which corresponds to the respective measurement protocol, which shows the respective examination area with or without the respective quantity of a contrast medium, which shows the respective examination object, and so on.

A semantic representation can be generated from the reference image in the same way as from the medical images of the first and the second training data set. The semantic representation is then entered into the trained machine learning model (optionally together with a semantic representation of a text prompt). The trained machine learning model generates a synthetic medical image based on the semantic representation(s) and noisy data.

A synthetic image can be generated based on several reference images. A semantic representation can be generated from each reference image. The semantic representations can be combined, e.g. through concatenation, average pooling, attention-weighted pooling and/or other combination methods. The combined semantic representation can be used as condition. This also applies analogously to other synthetic medical records (not just images). Synthetic medical records can be generated based on one or more reference medical records. A semantic representation is usually generated from each reference medical record. Several (i.e., more than one) semantic representations can be combined into a single semantic representation.

There are several options to combine multiple semantic representations into one semantic representation.

Multiple semantic representations can be combined into one semantic representation by concatenation, i.e., by sticking the semantic representations end-to-end. If the semantic representations are vectors, a longer vector or a matrix can be generated by concatenation. If the semantic representations are matrices, a matrix with more rows or columns or a tensor can be generated by concatenation. This method (concatenation) retains all original information but may result in a high-dimensional conditional input.

Multiple semantic representations can be combined into one semantic representation by summation, i.e., by summing the semantic representations together elementwise.

Multiple semantic representations can be combined into one semantic representation by performing a principal component analysis (PCA), and generating an embedding based on identified principle components.

Multiple semantic representations can be combined into one semantic representation by averaging, i.e., by taking the element-wise mean (e.g., arithmetic means) of the image embeddings.

Multiple semantic representations can be combined into one embedding by weighted averaging. Weighted averaging is similar to averaging but each semantic representation and/or each dimension of a semantic representation is assigned a weight before averaging. The weights can be determined based on the importance of each semantic representation and/or dimension, for example. The weights can be learned, for example. It is possible that the image encoder or feature encoder or a downstream artificial neural network that combines the semantic representations is included in the training of the generative model and that the attention weights are learned during the training.

It is possible that a feature encoder is used for generation semantic representations, the feature encoder comprising a CNN. Parameters of the CNN may be learned during the training of the generative model. For example, the CNN may perform a 1D convolution over the elements of the semantic representations and thus merge the semantic representations into a semantic representation.

The one or more synthetic medical records (e.g., synthetic medical images) generated by the trained generative model can be outputted (e.g., displayed on a display and/or printed on a printer), stored in a data storage, transmitted to a separate computer system, and/or used for training another machine learning model.

The aspects of the present disclosure are explained in more detail below with reference to examples and drawings, without wishing to limit the disclosure to the examples or the features and combinations of features shown in the drawings. If the same reference signs are used in different drawings, they have the same meaning.

For the sake of simplicity, the aspects of the present disclosure are explained in more detail in the drawings using medical images as medical records, without wishing to limit the disclosure to this embodiment.

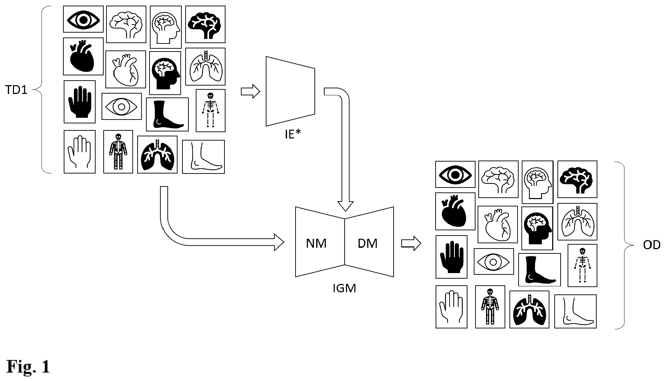

Fig. 1 shows schematically by way of example the first phase of training the generative model of the present disclosure.

In the example shown in Fig. 1 the generative model is an image generation model IGM.

In the first phase, the image generation model IGM is trained on a first training data set TD1. The first training data set TD1 comprises a variety of non-annotated medical images.

The image generation model IGM is a conditional diffusion model, comprising a noising model NM (encoder) and a denoising model DM (decoder).

The image generation model IGM is configured and trained to reconstruct each medical image of the first training data set TD 1.

In other words, the image generation model IGM is configured to receive a medical image, apply noise to the image (or a latent representation of the image in case the image generation model is a latent diffusion model) in a defined number of steps, and reconstruct the medical image (or the latent representation thereof) from the noisy data and output it as output data OD.

The reconstruction of the medical image is based on a condition. The condition is a semantic representation of the medical image. The semantic representation of the medical image is generated with a pre-trained image encoder IE*. The asterisk * in the reference sign IE* indicates that the pre-trained encoder IE* is not trained when the image generation model IGM is trained. However, it is generally possible to include the pre-trained image encoder IE* in the training. For example, it is possible to first train the image generation model IGM and have the parameters of the pre-trained image encoder IE* locked and then, in a further step, extend the training to the pre-trained image encoder IE* in an end-to-end training process. Other training procedures are also conceivable.

As described in this disclosure, the image encoder IE* may be an encoder of an autoencoder or an image encoder of a vision transformer or an image encoder of a CLIP model or another encoder that generates a semantic representation of an image (see above).

It is also possible to use several (different) image encoders that generate different semantic representations. The different semantic representations can be combined with each other to form a condition (e.g., by concatenation or pooling).

Fig. 2 shows schematically by way of example the second phase of training the generative model of the present disclosure.

As shown in Fig. 2, the trained image generation model IGM* from Fig. 1 was provided with a control model CM.

The control model copys the weights of neural network blocks of the trained image generation model IGM* into a "locked" copy and a "trainable" copy. The "trainable" copy learns a further condition. The "locked" copy preserves the trained image generation model IGM*. Details are shown in Figs. 3 (a), 3 (b), and 3 (c).

The control model CM added to the image generation model IGM* is trained with a second training dataset TD2. The second training dataset TD2 contains a plurality of annotated medical images I1, I2, I3, I4, I5. As shown in Fig. 2, an annotation A1, A2, A3, A4, A5 is available for each medical image I1, I2, I3, I4, I5. Usually, the number of annotated medical images is much larger than shown in Fig. 2. For example, there may be more than 100 annotated medical images.

The control model CM added to the image generation model IGM* is configured and trained to reconstruct each medical image.

During training, a medical image is fed to the image encoder IE*, in this example the medical image I5. The image encoder IE* generates a semantic representation of the medical image based on the medical image and passes this on as a condition to the trained image generation model IGM*, which is equipped with the control model CM.

In addition, the medical image (in this example the medical image I5) is fed to the control model CM added to the image generation model IGM*.

In addition, from the annotation of the medical image a semantic annotation representation is generated by means of a feature encoder FE* (in this example, a semantic annotation representation is generated from annotation A5 of the medical image I5). The semantic annotation representation is added to the control model CM as a further condition.

The trained image generation model IGM* equipped with the control model CM generates a reconstructed medical image (in this example the reconstructed medical image I5^{R}) as output data OD.

Figs. 3 (a), 3 (b), and 3 (c) show how a control model may be added to a pre-trained generative model.

The control model CM injects an additional condition c into neural network blocks nnb* of the image generation model IGM*.

Fig. 3 (a) shows a neural network block nnb* of a trained image generation model IGM*. The term "neural network block" refers to a set of neural layers that are commonly put together to form a single unit of a neural network, e.g., a residual neural network block, a convolution - batch normalization - ReLU block, a multi-head attention block, a transformer block, and/or other/further blocks.

The neural network block nnb* is configured to transform an input feature map *x* into another feature map*y*.

In Fig. 3 (b) a control model CM is added to the neural network block nnb* of the trained image generation model IGM*. To add a control model CM to such a trained neural network block nnb*, the parameters of the neural network block nnb* are locked and a trainable copy tc of the neural network block nnb* is generated.

The trainable copy tc takes a condition c (e.g., in the form of a vector) as input.

The trainable copy tc and the locked neural network block nnb* are connected, e.g., with convolution layers cl, with weights initialized to zeros so that they progressively change during the training. Such an architecture ensures that harmful noise is not added to the deep features of the trained image generation model at the beginning of the training and protects the pretrained backbone in the trainable copy tc from being damaged by such noise.

Fig. 3 (c) shows the addition of a control model CM to a trained image generation model IGM* using the example of a conditional diffusion model.

The trained image generation model IGM* as shown in Fig. 3 (c) is essentially a U-net with an encoder, a middle block, and a skip-connected decoder. Both the encoder and decoder contain 12 blocks, and the full model contains 25 blocks, including the middle block. Of the 25 blocks, 8 blocks may be down-sampling or up-sampling convolution layers, while the other 17 blocks may be main blocks that each contain 4 residual neural network layers and 2 vision transformers. For example, in Fig. 3 (c), the block A* may contain 4 residual neural network layers and 2 vision transformers, while the "×3" indicates that this block is repeated three times.

The control model CM is applied to each encoder level of the U-net. In particular, a trainable copy of the 12 encoding blocks and 1 middle block is created. The 12 encoding blocks may in 4 resolutions (e.g., 64 × 64, 32 × 32, 16 × 16, 8 × 8) with each one replicated 3 times. The outputs are added to the 12 skip-connections and 1 middle block of the U-net.

Given an input image I, image diffusion algorithms progressively add noise to the image and produce a noisy image. Given a set of conditions including time step (not shown in Fig. 3 (c)), a condition generated by feature encoder FE*, as well as a semantic representation of the image I generated by the image encoder IE*, image diffusion algorithms learn a network to predict the noise added to the noisy image.

The architecture shown in Figures 3 (a), 3 (b) and 3 (c) is based on the architecture disclosed in: L. Zhang et al.: Adding Conditional Control to Text-to-Image Diffusion Models, arXiv:2302.05543v2.

It should be pointed out once again that the embodiments shown in the figures are merely possibilities for realizing the aspects of the present disclosure, many other possibilities are conceivable.

Fig. 4 shows a schematic example of how a semantic representation can be generated from a medical record. In the example shown in Fig. 4, the medical record is a medical image I.

The medical image I is split into fixed-size patches P1 to P9. Patch embeddings *a* to *i* are generated by flattening the patches P1 to P9 and mapping the flattened patches by linear projection to a dimension corresponding to the input dimension of the transformer T. Position embeddings 1 to 9 are added to the patch embeddings to retain positional information. The resulting sequence serves as input to the transformer T.

In the example shown, the sequence is preceded by an embedding * with the position 0, which can contain global information about the medical image I1, e.g. at what time and/or in which phase of an examination it was generated and/or which examination region it shows and/or the modality of the medical image and/or the measurement conditions under which it was generated.

It is possible that other/further embeddings are added to the sequence. For example, one or more learnable input tokens can be added, that the conditional generative model can use as "registers" (see, e.g., T. Darcet et al.: Vision Transformers Need Registers, arXiv:2309.16588v1).

The transformer T generates a semantic representation SR from the sequence.

This process for generating semantic representations of images is known and described, for example, in: A. Dosovitsky et al.: An Image is Worth 16x16 Words: Transformers for Image Recognition at Scale, arXiv:2010.11929v2). It can be applied to medical records with modalities other than images.

Fig. 5 shows a schematic example of how parts of a semantic representation can be masked. In the example shown, the patches P1, P3, P4 and P8 are masked. The grey values of the masked patches can be set to zero, for example. This also sets the corresponding image tokens of the semantic representation SR to zero. However, masking does not have to be performed at patch level; it is also possible to perform masking at image token level.

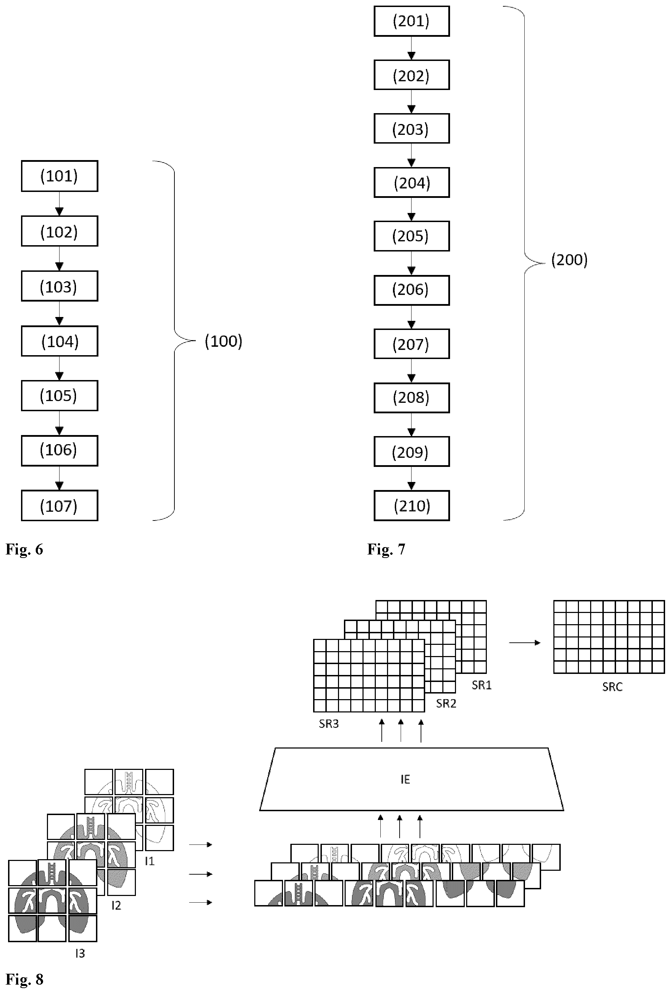

Fig. 6 shows an embodiment of the computer-implemented method of generating synthetic training data in form of a flow chart.

The method (100) comprises the step:

| | |
|---|---|
| (101) | providing a data set, the data set comprising a variety of non-annotated medical records, wherein each medical record of the variety of non-annotated medical records represents a patient or part thereof, |
| (102) | for each medical record of the variety of non-annotated medical records: generating a semantic representation of the medical record, |
| (103) | providing a generative model, |
| (104) | training the generative model in a self-supervised training procedure using the variety of non-annotated medical records and their semantic representations as training data, wherein the generative model is trained to reconstruct each medical record or a portion thereof using the respective semantic representation of the medical record as a conditioning input, |
| (105) | providing one or more prompts and/or reference medical records and/or noisy data, |
| (106) | generating one or more synthetic medical records based on the one or more prompts and/or reference medical records and/or noisy data using the trained generative model, |
| (107) | outputting the one or more synthetic medical records, storing the one or more synthetic medical records in a data storage, transmitting the one or more synthetic medical records to a separate computer system, and/or using the one or more synthetic medical records for training another machine learning model. |

Fig. 7 shows another embodiment of the computer-implemented method of generating synthetic training data in form of a flow chart.

The method (200) comprises the steps:

| | |
|---|---|
| (201) | providing a first data set, the first data set comprising a variety of non-annotated medical records, wherein each medical record of the variety of non-annotated medical records represents a patient or part thereof, |
| (202) | for each medical record of the variety of non-annotated medical records: generating a semantic representation of the medical record, |
| (203) | providing a generative model, |
| (204) | training the generative model in a self-supervised training procedure using the variety of non-annotated medical records and their semantic representations as first training data, wherein the generative model is trained to reconstruct each medical record or a portion thereof using the respective semantic representation of the medical record as a conditioning input, |
| (205) | adding a control model to the trained generative model, wherein the control model adds further condition control to the trained generative model, |
| (206) | providing a second data set, the second data set comprising a plurality of annotated medical records, wherein an annotation is available for each annotated medical record, |
| (207) | training the control model added to the trained generative model on the second data set, wherein the annotations are used as conditions in reconstruction of the annotated medical records, |
| (208) | providing one or more prompts and/or reference medical records and/or noisy data, |
| (209) | generating one or more synthetic medical records based on the one or more prompts and/or reference medical records and/or noisy data using the trained generative model and the trained control model, |
| (210) | outputting the one or more synthetic medical records, storing the one or more synthetic medical records in a data storage, transmitting the one or more synthetic medical records to a separate computer system, and/or using the one or more synthetic medical records for training another machine learning model. |

Fig. 8 shows a schematic example of the generation of several semantic representations and the combination of the semantic representations into a single semantic representation.

Fig. 8 shows three images I1, I2, and I3.

Patches are generated from each of the three images and fed to the image encoder IE. The image encoder IE generates a first semantic representation SR1 based on the patches of the first medical image I1, a second semantic representation SR2 based on the patches of the second medical image I2, and a third semantic representation SR3 based on the third medical image I3. The semantic representations can be generated as described in relation to Fig. 4.

The semantic representations SR1, SR2 and SR3 are combined into a single semantic representation SRC. This combined semantic representation SRC can be used as a condition.

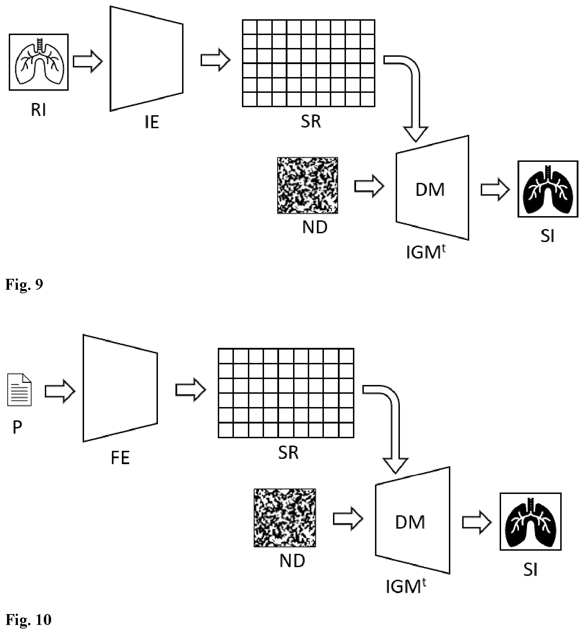

Fig. 9 shows schematically an embodiment of the method of generating synthetic training data using the trained generative model.

A trained image generation model IGM^{t} is used to generate the synthetic image SI. The trained image generation model IGM¹ may have been trained as described in relation to Figs. 1, 2, 3 (a), 3 (b), 3 (c), 4, and/or 5.

A reference image RI is provided. The reference image RI is fed to the image encoder IE. The image encoder IE generates a semantic representation SR based on the reference image RI. Noisy data ND is provided. The noisy data ND is fed to the denoising model DM of the trained image generation model IGMt. Similarly, the semantic representation SR is fed into the trained image generation model IGM^{t}. The trained image generation model IGM^{t} generates the synthetic image SI based on the noisy data ND and based on the semantic representation SR as a condition.

Fig. 10 shows schematically another embodiment of the method of generating synthetic training data using the trained generative model.

A trained image generation model IGM^{t} is used to generate the synthetic image SI. The trained image generation model IGM¹ may have been trained as described in relation to Figs. 1, 2, 3 (a), 3 (b), 3 (c), 4, and/or 5.

A text prompt P is provided. The text prompt P may specify what the synthetic image SI should show and/or what the synthetic image SI should look like and/or what format the synthetic image SI should have. The text prompt P is fed to a feature encoder FE. The feature encoder FE can be a text encoder, for example. The feature encoder FE generates a semantic representation SR based on the text prompt. Noisy data ND is provided. The noisy data ND is fed to the denoising model DM of the trained image generation model IGMt. Similarly, the semantic representation SR is fed into the trained image generation model IGM^{t}. The trained image generation model IGM¹ generates the synthetic image SI based on the noisy data ND and based on the semantic representation SR as a condition.

The operations in accordance with the teachings herein may be performed by at least one computer specially constructed for the desired purposes or general-purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer or processor. The term processor includes a single processing unit or a plurality of distributed or remote such units.

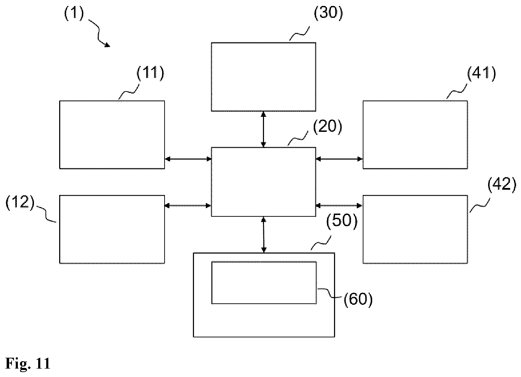

Fig. 11 illustrates a computer system (1) according to some example implementations of the present disclosure in more detail. The computer may include one or more of each of a number of components such as, for example, processing unit (20) connected to a memory (50) (e.g., storage device).

The processing unit (20) may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory (50) of the same or another computer.

The processing unit (20) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

## Claims

1. A computer-implemented method of generating synthetic training data, the method comprising the steps:
- providing a data set, the data set comprising a variety of non-annotated medical records, wherein each medical record of the variety of non-annotated medical records represents a patient or part thereof,
- for each medical record of the variety of non-annotated medical records: generating a semantic representation of the medical record,
- providing a generative model,
- training the generative model in a self-supervised training procedure using the variety of non-annotated medical records and their semantic representations as training data, wherein the generative model is trained to reconstruct each medical record or a portion thereof using the respective semantic representation of the medical record as a conditioning input,
- providing one or more prompts and/or reference medical records and/or noisy data,
- generating one or more synthetic medical records based on the one or more prompts and/or reference medical records and/or noisy data using the trained generative model,
- outputting the one or more synthetic medical records, storing the one or more synthetic medical records in a data storage, transmitting the one or more synthetic medical records to a separate computer system, and/or using the one or more synthetic medical records for training another machine learning model.

2. The method of claim 1, the method comprising:
- providing a first data set, the first data set comprising a variety of non-annotated medical records, wherein each medical record of the variety of non-annotated medical records represents a patient or part thereof,
- for each medical record of the variety of non-annotated medical records: generating a semantic representation of the medical record,
- providing a generative model,
- training the generative model in a self-supervised training procedure using the variety of non-annotated medical records and their semantic representations as first training data, wherein the generative model is trained to reconstruct each medical record or a portion thereof using the respective semantic representation of the medical record as a conditioning input,
- adding a control model to the trained generative model, wherein the control model adds further condition control to the trained generative model,
- providing a second data set, the second data set comprising a plurality of annotated medical records, wherein an annotation is available for each annotated medical record,
- training the control model added to the trained generative model on the second data set, wherein the annotations are used as conditions in reconstruction of the annotated medical records,
- providing one or more prompts and/or reference medical records and/or noisy data,
- generating one or more synthetic medical records based on the one or more prompts and/or reference medical records and/or noisy data using the trained generative model and the trained control model,
- outputting the one or more synthetic medical records, storing the one or more synthetic medical records in a data storage, transmitting the one or more synthetic medical records to a separate computer system, and/or using the one or more synthetic medical records for training another machine learning model.

3. The method of any one of claims 1 or 2, wherein each medical record is or comprises a medical image of a patient or a part thereof.

4. The method of any one of claims 2 to 3, wherein each annotation provides information about what is contained in the medical record and/or which patient and/or which part of a patient the medical record represents and/or what the medical record represents and/or how the medical record was created/acquired and/or other/additional information about the medical record and/or the content of the medical record and/or the creation/acquisition of the medical record.

5. The method of any one of claims 2 to 4, wherein each medical record is or comprises a first medical image of a patient or a part thereof of a first modality and each annotation of the medical record is or comprises a second medical image of the respective patient or the part thereof of a second modality, wherein the first modality and the second modality are different.

6. The method of any one of claims 2 to 5, wherein each medical record is or comprises a first radiologic image, the first radiologic image representing an examination area of a patient after application of a first amount of a contrast agent, and wherein each annotation is or comprises a second radiologic image of the respective patient, the second radiologic image representing the examination area of the respective patient after application of a second amount of a contrast agent, wherein the first amount and the second amount are different.

7. The method of any one of claims 2 to 6, wherein each medical record is or comprises a first radiologic image, the first radiologic image representing an examination area of a patient at a first time point before or after application of a contrast agent, and wherein each annotation is or comprises a second radiologic image of the respective patient, the second radiologic image representing the examination area of the respective patient at a second time point before or after application of the contrast agent, wherein the first time point and the second time point are different.

8. The method of any one of claims 1 or 2, wherein each medical record is or comprises a signal caused by a body action.

9. The method of any one of claims 1 to 8, wherein each semantic representation of each medical record is generated using a pre-trained machine learning model.

10. The method of claim 9, wherein the pre-trained machine learning model is or comprises a transformer.

11. The method of any one of claims 2 to 10, further comprising:
- generating a semantic representation of each annotation using a pre-trained machine learning model, wherein the semantic representation of an annotation is used as condition in reconstruction of the respective annotated medical record.

12. The method of claim11, wherein the pre-trained machine learning model is or comprises a transformer.

13. The method of any one of claims 1 to 12, wherein the generative model is a conditional diffusion model.

14. A computer system comprising:
a processor; and
a memory storing an application program configured to perform, when executed by the processor, an operation, the operation comprising:
- providing a data set, the data set comprising a variety of non-annotated medical records, wherein each medical record of the variety of non-annotated medical records represents a patient or part thereof,
- for each medical record of the variety of non-annotated medical records: generating a semantic representation of the medical record,
- providing a generative model,
- training the generative model in a self-supervised training procedure using the variety of non-annotated medical records and their semantic representations as training data, wherein the generative model is trained to reconstruct each medical record or a portion thereof using the respective semantic representation of the medical record as a conditioning input,
- providing one or more prompts and/or reference medical records and/or noisy data,
- generating one or more synthetic medical records based on the one or more prompts and/or reference medical records and/or noisy data using the trained generative model,
- outputting the one or more synthetic medical records, storing the one or more synthetic medical records in a data storage, transmitting the one or more synthetic medical records to a separate computer system, and/or using the one or more synthetic medical records for training another machine learning model.

15. A non-transitory computer readable storage medium having stored thereon software instructions that, when executed by a processor of a computer system, receiving a set of class-labelled medical images,
- providing a data set, the data set comprising a variety of non-annotated medical records, wherein each medical record of the variety of non-annotated medical records represents a patient or part thereof,
- for each medical record of the variety of non-annotated medical records: generating a semantic representation of the medical record,
- providing a generative model,
- training the generative model in a self-supervised training procedure using the variety of non-annotated medical records and their semantic representations as training data, wherein the generative model is trained to reconstruct each medical record or a portion thereof using the respective semantic representation of the medical record as a conditioning input,
- providing one or more prompts and/or reference medical records and/or noisy data,
- generating one or more synthetic medical records based on the one or more prompts and/or reference medical records and/or noisy data using the trained generative model,
- outputting the one or more synthetic medical records, storing the one or more synthetic medical records in a data storage, transmitting the one or more synthetic medical records to a separate computer system, and/or using the one or more synthetic medical records for training another machine learning model.
